(19) Europäisches Patentamt
European Patent Office
Office européen des brevets

(11) EP 1 308 500 A1

# (12) EUROPEAN PATENT APPLICATION

(43) Date of publication:
**07.05.2003 Bulletin 2003/19**

(51) Int Cl.[7]: **C11D 1/722**, C11D 1/44, C11D 3/20, C11D 3/33

(21) Application number: **02023548.7**

(22) Date of filing: **22.10.2002**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **31.10.2001 JP 2001334166**

(71) Applicant: **Menicon Co., Ltd.**
**Nagoya-shi Aichi (JP)**

(72) Inventors:
- **Tsuzuki, Akira**
  **Nagoya-shi, Aichi (JP)**
- **Tanikawa, Sadayasu**
  **Kasugai-shi, Aichi (JP)**
- **Ochi, Takahiro**
  **Kasugai-shi, Aichi (JP)**

(74) Representative: **Hartz, Nikolai F., Dr. et al**
**Wächtershäuser & Hartz,**
**Patentanwälte,**
**Tal 29**
**80331 München (DE)**

## (54) Ophthalmic composition

(57) An ophthalmic composition which contains a component A consisting of at least one member selected from the group consisting of polyoxyalkylene block copolymers and their derivatives, and a component B consisting of at least one member selected from the group consisting of caproic acid and its derivatives.

EP 1 308 500 A1

Printed by Jouve, 75001 PARIS (FR)

## Description

The present invention relates to an ophthalmic composition, particularly an ophthalmic composition having an excellent cleaning effect and in addition, an adequate safety for the eye, and specifically it relates to an ophthalmic solution such as eye drops and an agent for contact lenses such as a cleaning solution for contact lenses.

Conventionally, contact lenses have been classified into water-nonabsorptive contact lenses and water-absorptive contact lenses, and classified into hard contact lenses and soft contact lenses. On each of these contact lenses, a stain of e.g. lipids (eye lipids) derived from the tear may be deposited when the lens is put on the eye in some cases, and such a stain on the lens due to the tear may cause deterioration in comfortableness in wearing or eye problems such as failure of eyesight or congestion of cornea, and accordingly it is essential to apply a cleaning treatment to a contact lens in order to safely and comfortably use the contact lens every day.

For such a cleaning treatment of a contact lens, a proper cleaning solution for contact lenses having a cleaning or removing effect over a stain is usually used. As such a cleaning solution for contact lenses, various solutions having a surfactant as a cleaning component added and incorporated therein have been proposed, and one having a polyoxyalkylene block copolymer such as a polyoxyethylene-polyoxypropylene block copolymer or a derivative thereof incorporated may, for example, be known.

However, with respect to the cleaning solution for contact lenses containing a polyoxyalkylene block copolymer or its derivative as a cleaning component, importance is usually attached to safety for the eye, and the concentration of the cleaning component in the solution is suppressed to be as low as possible so that the standards for contact lenses such as diameter or base curve will not change and the shape or physical properties of the contact lenses will not be impaired. At such a low concentration of the cleaning component, no adequate cleaning power such as lipid-solubilizing power may be obtained. Accordingly, there is a fear that when a cleaning treatment of a contact lens is carried out by using such a cleaning solution, a stain of e.g. lipids tends to remain and be deposited on the contact lens, and the eye may harmfully be influenced.

On the other hand, caproic acid and its derivatives have conventionally been used as a medicine such as an anti-inflammatory agent or an antiallergic agent. For example, in JP-A-7-25755 which discloses a method for preparing an aqueous eye lotion containing solubilized vitamin A or in JP-A-8-198746 which discloses a solubilized eye drop, ε-aminocaproic acid which is a derivative of caproic acid is blended as an agent, as the case requires.

Further, not only ε-aminocaproic acid has been used as the above-described agent such as an anti-inflammatory agent or an antiallergic agent but also it has been incorporated into an aqueous solution for various purposes. Particularly, various solutions (solvents) having both ε-aminocaproic acid and a nonionic surfactant incorporated therein have been disclosed in JP-A-5-4918, JP-A-5-17350, JP-A-5-139955, JP-A-10-29937 and JP-A-11-228404. Specifically, for example, in JP-A-5-139955, ε-aminocaproic acid and a fat-soluble substance are used together for an instillation comprising an ester-based nonionic surfactant and glycyrrhizic acid salt with a purpose of suppressing hydrolysis of the surfactant in the solution to achieve stable appearance for a long time. Further, in JP-A-11-228404, ε-aminocaproic acid and a nonionic surfactant are used together for an aqueous eye drop comprising dipivefrin or a pharmaceutically acceptable salt thereof as an active ingredient with a purpose of significantly improving stability of dipivefrin in the vicinity of physiological pH.

Accordingly, the present inventors have conducted various studies on a solution comprising caproic acid or its derivative, and the above-described solution comprising both ε-aminocaproic acid and a nonionic surfactant, and as a result, they have found that not only the solution having caproic acid or its derivative alone incorporated therein but also the conventional solution comprising both ε-aminocaproic acid and a nonionic surfactant has a low or no lipid-solubilizing power, and their cleaning effect over a stain of lipids is inadequate.

Under these circumstances, the present inventors have further conducted extensive studies and as a result, found that the above caproic acid and its derivatives have an effect to significantly improve the lipid-solubilizing power which a nonionic surfactant particularly the above polyoxyalkylene block copolymer or its derivative possesses, and with a solution comprising at least one member selected from the group consisting of caproic acid and its derivatives, together with at least one member selected from the group consisting of polyoxyalkylene block copolymers and their derivatives, both useful cleaning effect over a stain of lipids and reduction in toxicity against the eye can be achieved.

Accordingly, the present invention has been accomplished on the basis of the above discoveries, and it is an object of the present invention to provide an ophthalmic composition which is excellent in a cleaning effect over a stain in the eye or a stain on a contact lens, particularly a stain of lipids, and with which safety for the eye can adequately be secured.

According to the present invention, there is provided an ophthalmic composition which contains a component A consisting of at least one member selected from the group consisting of polyoxyalkylene block copolymers and their derivatives, and a component B consisting of at least one member selected from the group consisting of caproic acid and its derivatives.

Now, the present invention will be described in detail with reference to the preferred embodiment.

In the ophthalmic composition of the present invention, at least one member selected from the group consisting of polyoxyalkylene block copolymers and their derivatives as the component A and at least one member selected from the group consisting of caproic acid and its derivatives as the component B are incorporated in combination as essential components, whereby the lipid-solubilizing power which the component A originally possesses is effectively reinforced. Accordingly, while securing adequate safety for the eye by properly adjusting the concentration of the component A, an excellent cleaning or removing effect over a stain in the eye or a stain on a contact lens, particularly a stain due to the tear attached to a contact lens, and particularly a stain of lipids, can advantageously be realized.

In a preferred embodiment of the ophthalmic composition according to the present invention, as the above component A, a polyoxyethylene-polyoxypropylene block copolymer or its derivative is advantageously used, and the polyoxyethylene-polyoxypropylene block copolymer or its derivative is preferably one having a HLB value of from 2 to 40.

In another preferred embodiment of the present invention, the above component A is contained preferably in a proportion of from 0.01 to 5.0 w/w%, and the above component B is contained preferably in a proportion of from 0.01 to 5.0 w/w%. By employing such contents, safety for the eye can advantageously be secured, and the cleaning effect over a stain of lipids can more advantageously be obtained.

Further, the ophthalmic composition of the present invention may further contain, in addition to the above components A and B, at least one member selected from the group consisting of a preservative, a thickener, a buffering agent, a chelating agent, an isotonicity agent and a surfactant as the case requires, and by addition of such an additive component, a further function depending upon the component such as a function of preserving a contact lens may advantageously be imparted.

Further, in another preferred embodiment of the present invention, the above ophthalmic composition has an adequate safety for the eye and can thereby be used as an ophthalmic solution. By using this, an effect of caproic acid or its derivative to prevent inflammation of the eye, as a therapeutic agent for e.g. inflammation or allergy, can be obtained, and in addition, a stain attached on the eye can advantageously be cleaned or removed. Further, the ophthalmic composition of the present invention may be administered to the eye having a contact lens put thereon, whereby the contact lens can be cleaned.

Further, in another preferred embodiment of the present invention, the above ophthalmic composition can preferably be used as an agent for contact lenses. Further, it is also preferred to use said agent for contact lenses as a cleaning solution for contact lenses for cleaning a contact lens taken off from the eye, by bringing the contact lens into contact with said cleaning solution for contact lenses.

The ophthalmic composition of the present invention contains the component A, as one of essential components, consisting of a polyoxyalkylene block copolymer or its derivative or a combination thereof, and thereby shows good surface active effect.

In the present invention, as the polyoxyalkylene block copolymer or its derivative as the component A, ophthalmologically acceptable known high-molecular compounds having a structure or a partial structure comprising two types of polyoxyalkylene groups, one showing hydrophilicity and the other showing lipophilicity, or more polyoxyalkylene groups, aligned and connected in a block, are advantageously employed. Among such high-molecular compounds, more preferred is one wherein each of the above at least two types of polyoxyalkylene groups has a carbon number of from 2 to 4, and particularly preferred is a polyoxyethylene-polyoxypropylene block copolymer or its derivative, which has a polyoxyethylene (POE) chain composed of a polyoxyethylene group and a polyoxypropylene (POP) chain composed of a polyoxypropylene group.

Here, as the polyoxyethylene-polyoxypropylene block copolymer, Pluronic, Pluronic R, Tetronic and Tetronic R (manufactured by BASF, Germany) which are commercially available as nonionic surfactants, and known polymers such as Poloxamer, may, for example, be employed, and among them, use of Poloxamer 124, Poloxamer 188, Poloxamer 237, Poloxamer 338, Poloxamer 407, Tetronic 904, Tetronic 908, Tetronic 1103 or Tetronic 1107 may be recommended.

As a specific example of the derivative of the polyoxyethylene-polyoxypropylene block copolymer used as the component A, a high-molecular compound obtained by etherification or esterification of a hydroxyl group on one or each terminal of a polyoxyethylene-polyoxypropylene block copolymer by a conventional modification means may be mentioned, and its representative examples include POE-POP type ones such as polyoxyethylene-polyoxypropylene monoalkyl ether, polyoxyethylene-polyoxypropylene dialkyl ether, polyoxyethylene-polyoxypropylene monoalkyl ester and polyoxyethylene-polyoxypropylene dialkyl ester, and POEx POP-POE type ones.

Here, among such polyoxyethylene-polyoxypropylene block copolymer derivatives, the POE-POP type one may be represented by any of the following general formulae (a) to (h) .

(a) $RO\text{-}(OE)_x\text{-}(OP)_y\text{-}H$
(b) $HO\text{-}(OE)_x\text{-}(OP)_y\text{-}R$
(c) $RCOO\text{-}(OE)_x\text{-}(OP)_y\text{-}H$

(d) HO-$(OE)_x$-$(OP)_y$-COR
(e) RO-$(OE)_x$-$(OP)_y$-R'
(f) RO-$(OE)_x$-$(OP)_y$-COR'
(g) RCOO-$(OE)_x$-$(OP)_y$-R'
(h) RCOO-$(OE)_x$-$(OP)_y$-COR'

In the above general formulae (a) to (h), OE is an oxyethylene group ($-CH_2CH_2O-$), OP is an oxypropylene group ($-CH_2CH(CH_3)O-$), and each of R and R' is a $C_{1-20}$ linear or branched alkyl group, and in the general formulae (e) to (h), R and R' may be the same or different. Further, x and y which are numbers of repetitions of the above OE and OP, are integers of from 5 to 100 and from 1 to 10, respectively.

Specific examples of the polyoxyethylene-polyoxypropylene block copolymer derivatives represented by the above general formulae (a) to (h) include POE(10)POP(4) monocetyl ether, POE(20)POP(4) monocetyl ether, POE (20)POP(8) monocetyl ether, POE(20)POP(6) decyl tetradecyl ether, POE(30)POP(6) decyl tetradecyl ether, POE(10) POP(4) monocetyl ester, POE(20)POP(4) monocetyl ester, POE(20)POP(8) monocetyl ester, POE(20)POP(6) decyl tetradecyl ester, POE(30)POP(6) decyl tetradecyl ester, POE(10)POP(4) monolauryl ether, POE(10)POP(4) monolauryl ester, POE(3)POP(1) cetyl acetate, POE(3)POP(1) isocetyl acetate, POE(3)POP(1) cetyl acetate and POE(3)POP (1) isocetyl acetate.

Further, examples of the POE-POP-POE type high-molecular compounds as representative examples of the polyoxyethylene-polyoxypropylene block copolymer derivatives, include compounds represented by the following general formulae (i) to (n).

(i) RO-$(OE)_a$-$(OP)_b$-$(OE)_a$-H
(j) RO-$(OE)_a$-$(OP)_b$-$(OE)_a$-R'
(k) RCOO-$(OE)_a$-$(OP)_b$-$(OE)_a$-H
(l) RCOO-$(OE)_a$-$(OP)_b$-$(OE)_a$-COR'
(m) OH-$(OE)_a$-$(OP)_b$-$(OE)_a$-R
(n) OH-$(OE)_a$-$(OP)_b$-$(OE)_a$-COR

In the above general formulae (i) to (n), each of OE, OP, R and R' is as defined for the above general formulae (a) to (h), and a and b which are numbers of repetitions of OE and OP, are integers of from 5 to 150 and from 10 to 100, respectively, and preferably a/b=0.5 to 3.5.

Further, other examples of the polyoxyethylene-polyoxypropylene block copolymer derivatives include poloxamine type high-molecular compounds which are tetrafunctional block copolymers obtained by sequentially adding ethylene oxide and propylene oxide to ethylene diamine, represented by the following general formulae (o) to (y) .

(o)
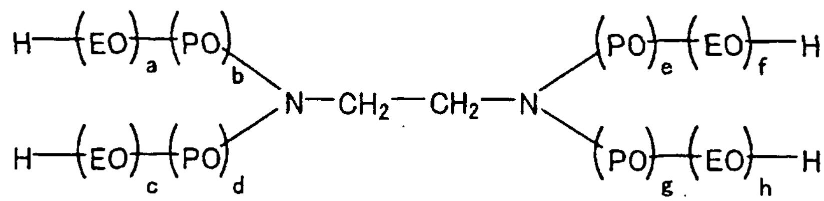

(p)
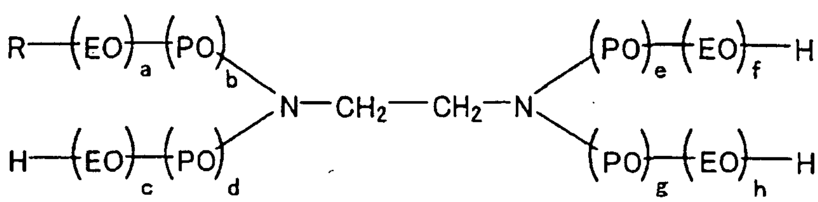

(q) 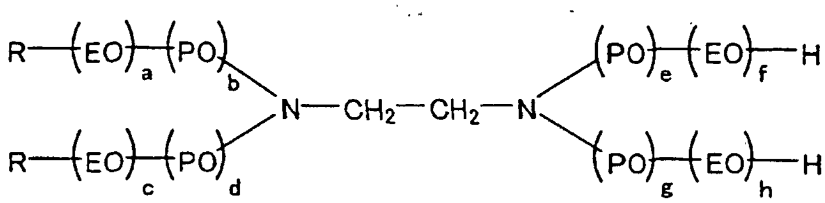

(r) 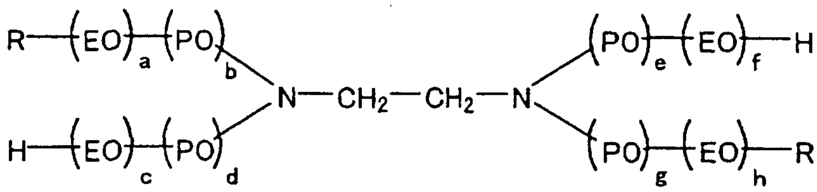

(s) 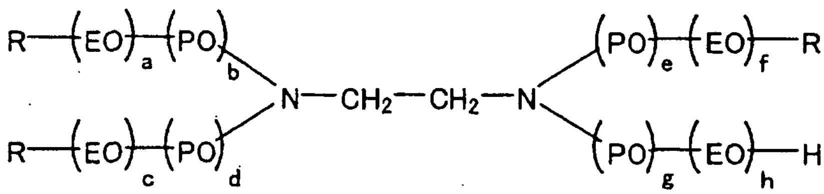

(t) 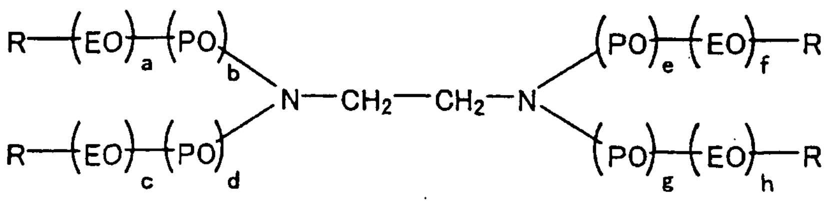

(u) 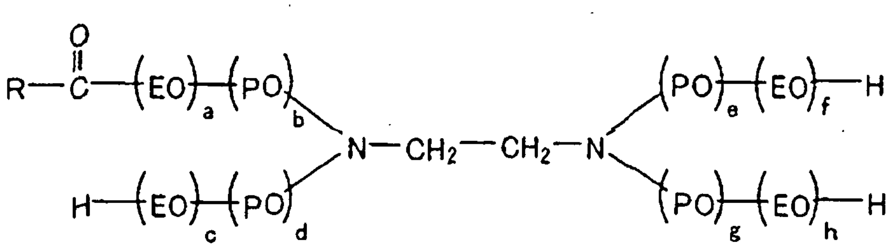

(v) 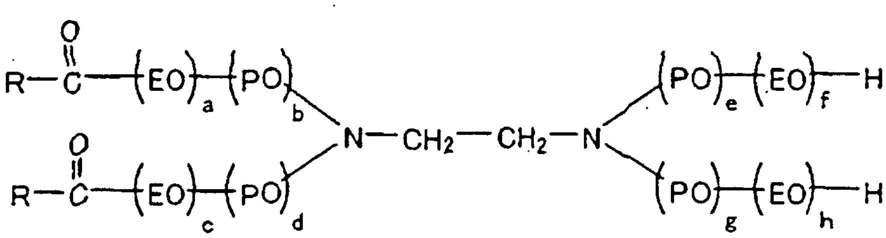

(w)

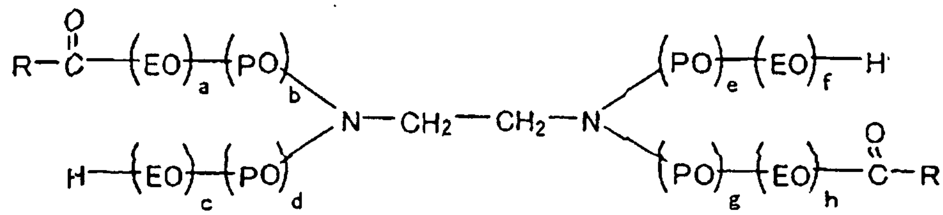

(x)

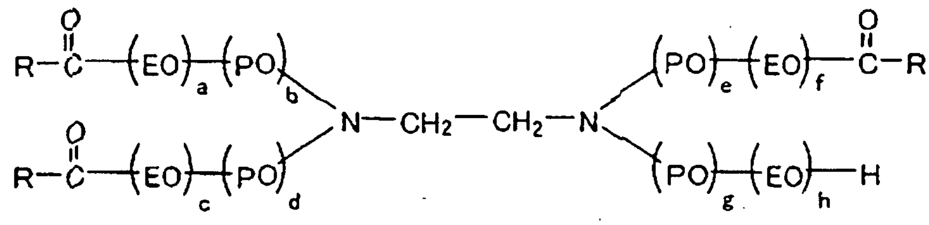

(y)

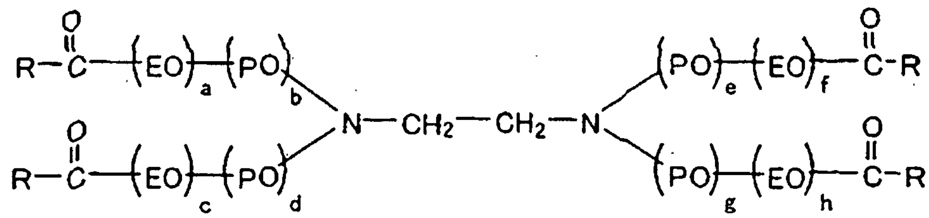

In the above general formulae (o) to (y), R is as defined above, each of a, c, f and h is an integer of from 1 to 150, and each of b, d, e and g is an integer of from 2 to 50, and preferably a/b=0.015 to 30.

In the present invention, the polyoxyethylene-polyoxypropylene block copolymer or its derivative preferably has a value of HLB (hydrophile-lipophile balance) of from 2 to 40, suitably from 7 to 30, in view of the purpose of the present invention of realizing an excellent cleaning effect.

In the ophthalmic composition of the present invention containing at least one member of such specific high-molecular compounds as the component A, at least one member selected from the group consisting of caproic acid and its derivatives as the component B coexists, whereby cleaning power, particularly lipid-solubilizing power, based on the surface active property of the component A is significantly reinforced, and accordingly a cleaning effect can be obtained even when the content of the component A is suppressed to a low concentration, specifically to a concentration of a level of 0.01 w/w%. Accordingly, in the present invention, the component A is used at a concentration of usually at least 0.01 w/w%, preferably at least 0.1 w/w%, more preferably at least 0.3 w/w%. However, if the content is too high, the eye may be irritated, the cytotoxicity tends to be high, and the safety for the body tends to decrease, and accordingly as the upper limit, the concentration is usually at most 5.0 w/w%, preferably at most 3.0 w/w%, more preferably at most 2.0 w/w%.

The caproic acid or its derivatives as the component B to be incorporated in the ophthalmic composition of the present invention together with the component A synergically improves cleaning power such as lipid-solubilizing power which the component A originally possesses, by the combination with the component A, whereby an excellent cleaning effect which can never be expected with the component A alone can be realized.

Examples of the caproic acid or its salt include ε-aminocaproic acid, methyl caproate, 2-fluoroethyl caproate, liexanyl caproate, ε-aminocaproic acid bioprase TCC, hexyl caproate, n-caproic acid and ethyl caproate, and one or a combination of at least two among these compounds may be used as the component B, and ε-aminocaproic acid, hydroxyprogesterone caproate or hydrocortisone caproate is advantageously used.

In the present invention, the amount of the component B may optionally be set taking e.g. the type of the high-molecular compound as the component A into consideration in order that the synergistic cleaning effect as the purpose of addition of the component B can advantageously be realized, and the component B is used at a concentration of usually from 0.01 to 5.0 w/w%, preferably from 0.03 to 4.0 w/w%, more preferably from 0.5 to 3.5 w/w%. However, if the amount of the component B is too small, no adequate effect of improving the cleaning power will be obtained, and on the contrary, if the amount is too large, the safety for the eye may be impaired, or the shape or physical properties

of a contact lens may be impaired such that standards for a contact lens such as a diameter or a base curve may be changed.

The ophthalmic composition of the present invention is prepared by adding and incorporating such specific two types of components A and B in proper amounts into a proper aqueous medium in an optional order in a conventional method. In the present invention, in addition to the components A and B, one or more additive components conventionally used for ophthalmic solutions and solutions for contact lenses may further be incorporated in a conventional amount as the case requires. Such an additive component is preferably one which has a high safety for the body, which is adequately acceptable ophthalmologically, and which has no influence over the shape or physical properties of a contact lens, and used preferably in an amount satisfying such essentialities, whereby a function depending upon the additive component can advantageously be imparted to the cleaning solution without impairing the effects of the present invention.

In order to advantageously impart disinfection effect on the eye or a contact lens, and preservative and preservation effects on the ophthalmic composition, a preservative having a preservative effect may be added to the ophthalmic composition of the present invention. As such a preservative, a proper one may be selected from known preservatives, and used alone or in combination as a mixture of at least two.

As the preservative, sorbic acid, potassium sorbate, benzoic acid or its salt, ethyl parahydroxybenzoate, butyl parahydroxybenzoate, propyl parahydroxybenzoate, methyl parahydroxybenzoate or chlorobutanol may, for example, be mentioned.

Further, in the present invention, a thickener may be added so as to properly adjust the viscosity of the ophthalmic composition, and examples of the thickener include gums such as heteropolysaccharides; synthetic organic high-molecular compounds such as polyvinyl alcohol, poly-N-vinyl pyrrolidone, polyethylene glycol, polypropylene glycol and polyacrylamide; cellulose derivatives such as hydroxyethyl cellulose, hydroxypropyl methylcellulose, carboxymethyl cellulose and methyl cellulose; and starch derivatives.

Further, with respect to the ophthalmic composition of the present invention, if the pH value or the osmotic pressure is too high or too small, there is a fear that the eye may be irritated or eye problems may be caused, and accordingly the pH value of the cleaning solution is preferably adjusted within a range of from about 5.3 to about 8.5 by addition of a pH adjustor, and the osmotic pressure is preferably adjusted within a range of from about 200 to about 400 mOsm/kg by addition of an isotonicity agent. As the pH adjustor to be used for adjustment of pH, e.g. sodium hydroxide or hydrochloric acid may be used, and as the isotonicity agent to be used for adjustment of the osmotic pressure, at least one compound selected from the group consisting of sodium chloride, potassium chloride, saccharides, sugar alcohols, and polyhydric alcohols and their ethers and esters, is usually used.

In order to keep the pH of the ophthalmic composition within the above effective and ophthalmologically safe range, usually at least one buffering agent may be added. As the buffering agent, a conventionally known one may optionally be selected and used. Specifically, acids such as phosphoric acid, boric acid, carboxylic acid and oxycarboxylic acid, salts thereof (such as sodium salt), and further, Good-Buffer, tris(hydroxymethyl)aminomethane (Tris), bis(2-hydroxyethyl)iminotris(hydroxymethyl)methane (Bis-Tris) and sodium hydrogen carbonate, may, for example, be mentioned from the viewpoint that they have safety for the eye and influences over a contact lens can be minimized.

Further, there is a possibility that e.g. calcium as a stain from the tear may be deposited or adsorbed on a contact lens, particularly on a soft contact lens in general, and accordingly a chelating agent may advantageously be added to the ophthalmic composition to prevent such inconvenience. As the chelating agent, ethylenediaminetetraacetic acid (EDTA) or its salts, such as disodium ethylenediaminetetraacetate (EDTA·2Na) or trisodium ethylenediaminetetraacetate (EDTA·3Na), may, for example, be used.

Further, in the ophthalmic composition according to the present invention, a known surfactant, advantageously a surfactant other than the above component A, may be added and incorporated so long as it does not inhibit the synergistic effect caused by the combination of the components A and B, in an amount not impairing the effect, whereby the cleaning effect over lipids will be more excellent. As the surfactant, preferred is a polyethylene glycol derivative such as a polyoxyethylene alkylphenyl ether formaldehyde condensate represented by tyloxapol, a sorbitan fatty acid ester such as sorbitan monooleate represented by polysorbate 80 or sorbitan sesquioleate, a polyoxyethylene sorbitan fatty acid ester such as sorbitan polyoxyethylene monooleate, a glycerol fatty acid ester such as glyceryl monostearate, a polyethylene glycol fatty acid ester such as polyethylene glycol monostearate, a polyoxyethylene alkyl ether such as polyoxyethylene lauryl ether, or polyoxyethylene castor oil, in view of safety for the body.

As evident from the above explanation, as the aqueous medium to which the above components A and B and another component are added to prepare the ophthalmic composition of the present invention, in addition to water itself such as running water, purified water or distilled water, physiological saline or a sodium chloride-containing aqueous solution, or a known cleaning solution for contact lenses or an ophthalmic solution such as eye drops, may, for example, be employed so long as it is a solution consisting essentially of water.

Further, the ophthalmic composition of the present invention has a high lipid-solubilizing power as disclosed hereinafter, in which even a water-insoluble substance which is hardly dissolved in an aqueous medium can easily be

dissolved. By utilizing such a nature, it is possible to further add a water-insoluble substance which has conventionally been employed for the ophthalmic composition in addition to the above components. Such a water-insoluble substance may, for example, be vitamin such as vitamin A (including retinol palmitate, β-carotene, etc.) or vitamin E (d-α-tocopherol acetate, etc.), or a refrigerant such as menthol, borneol, campher, graniol, eucalyptus oil, bergamot oil, fennel oil, peppermint oil, rose oil or COOLMINT.

When a contact lens is cleaned by using the ophthalmic composition of the present invention thus obtained, an optional means may be employed, such as a means of using said ophthalmic composition as a cleaning solution for contact lenses, and soaking a contact lens taken off from the eye in the cleaning solution for contact lenses of the present invention filled in a proper container for a predetermined time, or cleaning the contact lens with said solution by rubbing, or a means of using said ophthalmic composition as an ophthalmic solution and administering it in a proper amount to the eye having a contact lens put thereon so that the contact lens is brought into contact with the ophthalmic composition on the eye and is cleaned. Here, in a case where the ophthalmic composition of the present invention is used as an ophthalmic solution, the eye, in addition to the contact lens, is cleaned of course.

Accordingly, when the eye or contact lens is cleaned by the ophthalmic composition of the present invention, a stain due to the tear such as lipids attached to the eye or contact lens can effectively be removed. Further, as the ophthalmic composition of the present invention has a high safety for the eye, no eye problems or the like will be caused even when the cleaning treatment of a contact lens by soaking or rubbing is carried out for a long period of time, and further when the ophthalmic composition is administered to the eye so as to clean the contact lens on the eye. Still further, the contact lenses are not impaired by the ophthalmic composition of the present invention.

Further, needless to say, the contact lens cleaned on the eye or in a state where it is taken off from the eye, can be subjected to a sterilization treatment by e.g. a sterilization solution for contact lenses prepared separately as the case requires.

Further, a contact lens to which the ophthalmic composition of the present invention is applied is not particularly limited, and the ophthalmic composition applies to all types of soft contact lenses including a low water-absorptive type and a high water-absorptive type, and hard contact lenses, and e.g. the material of a contact lens is not limited.

Now, the present invention will be explained in further detail with reference to Examples and Comparative Examples. However, it should be understood that the present invention is by no means restricted to such specific Examples. Further, various changes, modifications or improvements may be made to the present invention other than the following Examples or the above specific description by those skilled in the art without departing from the scope of the present invention.

Predetermined additive components were added to purified water in various proportions as identified in the following Tables 1 to 3 to prepare various sample solutions having a pH of 6.8 (sample Nos. 1-36). For the preparation of the sample solutions, Poloxamer 407 (Px 407, manufactured by BASF, molecular weight: 8,000 to 12,000), Tetronic 1107 (manufactured by BASF, average molecular weight: about 15,000) or polyoxyethylene(20)polyoxypropylene(4) cetyl ether (PBC-34, manufactured by Nikko Chemicals Co., Ltd.), as a polyoxyalkylene block copolymer or its derivative (component A) as an essential additive component in the present invention, and ε-aminocaproic acid (manufactured by Daiichi Pure Chemicals Co., Ltd.) as caproic acid or its derivative (component B) as the other essential additive component, were used. Further, as a surfactant other than the component A, polyoxyethylene(20) sorbitan monooleate (Tween 80, manufactured by Nikko Chemicals Co., Ltd., TO-10M), polyoxyethylene nonyl phenyl ether (NP-18TX, manufactured by Nikko Chemicals Co., Ltd.) or tyloxapol (4-(1,1,3,3-tetramethylbutyl)phenol polymer with formaldehyde and oxirane, manufactured by Aldrich, U.S.A.) was used. Further, as other additive components, hydroxypropyl methylcellulose (HPMC2910, manufactured by Shin-Etsu Chemical Co., Ltd.) as a thickner, disodium ethylenediaminetetraacetate (EDTA·2Na, manufactured by Teikoku Kagaku Sangyo K.K.) as a chelating agent, boric acid and sodium borate as buffering agents and sodium chloride and potassium chloride as isotonicity agents were used. Each of the obtained sample solutions was subjected to the following test on cleaning effect over lipids, test on adjustability to lenses and test on protein deposit inhibitory effect.

Table 1

| Additive components | Sample No. | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| Px 407 | 0.5 | - | - | - | - | - | 0.5 | - | - | - | - | - |
| Tetronic 1107 | - | 0.5 | - | - | - | - | - | 0.5 | - | - | - | - |
| PBC-34 | - | - | 0.5 | - | - | - | - | - | 0.5 | - | - | - |
| Tween 80 | - | - | - | 0.5 | - | - | - | - | - | 0.5 | - | - |
| NP-18TX | - | - | - | - | 0.5 | - | - | - | - | - | 0.5 | - |
| Tyloxapol | - | - | - | - | - | 0.5 | - | - | - | - | - | 0.5 |
| ε-aminocaproic acid | 0.1 | 0.1 | 0.1 | 0.1 | 0.1 | 0.1 | 0.5 | 0.5 | 0.5 | 0.5 | 0.5 | 0.5 |
| HPMC 2910 | 0.15 | 0.15 | 0.15 | 0.15 | 0.15 | 0.15 | 0.15 | 0.15 | 0.15 | 0.15 | 0.15 | 0.15 |
| EDTA·2Na | 0.05 | 0.05 | 0.05 | 0.05 | 0.05 | 0.05 | 0.05 | 0.05 | 0.05 | 0.05 | 0.05 | 0.05 |
| Potassium sorbate | 0.15 | 0.15 | 0.15 | 0.15 | 0.15 | 0.15 | 0.15 | 0.15 | 0.15 | 0.15 | 0.15 | 0.15 |
| Boric acid | 0.5 | 0.5 | 0.5 | 0.5 | 0.5 | 0.5 | 0.5 | 0.5 | 0.5 | 0.5 | 0.5 | 0.5 |
| NaCl | 0.48 | 0.48 | 0.48 | 0.48 | 0.48 | 0.48 | 0.42 | 0.42 | 0.42 | 0.42 | 0.42 | 0.42 |
| KCl | 0.1 | 0.1 | 0.1 | 0.1 | 0.1 | 0.1 | 0.08 | 0.08 | 0.08 | 0.08 | 0.08 | 0.08 |

Addition proportion (unit):w/w%

Table 2

| Additive components | Sample No. | | | | | | | | | | | |
|---|---|---|---|---|---|---|---|---|---|---|---|---|
| | 13 | 14 | 15 | 16 | 17 | 18 | 19 | 20 | 21 | 22 | 23 | 24 |
| Px 407 | 0.5 | - | - | - | - | - | 0.5 | - | - | - | - | - |
| Tetronic 1107 | - | 0.5 | - | - | - | - | - | 0.5 | - | - | - | - |
| PBC-34 | - | - | 0.5 | - | - | - | - | - | 0.5 | - | - | - |
| Tween 80 | - | - | - | 0.5 | - | - | - | - | - | 0.5 | - | - |
| NP-18TX | - | - | - | - | 0.5 | - | - | - | - | - | 0.5 | - |
| Tyloxapol | - | - | - | - | - | 0.5 | - | - | - | - | - | 0.5 |
| ε-aminocaproic acid | 1 | 1 | 1 | 1 | 1 | 1 | 3 | 3 | 3 | 3 | 3 | 3 |
| HPMC 2910 | 0.15 | 0.15 | 0.15 | 0.15 | 0.15 | 0.15 | 0.15 | 0.15 | 0.15 | 0.15 | 0.15 | 0.15 |
| EDTA·2Na | 0.05 | 0.05 | 0.05 | 0.05 | 0.05 | 0.05 | 0.05 | 0.05 | 0.05 | 0.05 | 0.05 | 0.05 |
| Potassium sorbate | 0.15 | 0.15 | 0.15 | 0.15 | 0.15 | 0.15 | 0.15 | 0.15 | 0.15 | 0.15 | 0.15 | 0.15 |
| Boric acid | 0.5 | 0.5 | 0.5 | 0.5 | 0.5 | 0.5 | 0.5 | 0.5 | 0.5 | 0.5 | 0.5 | 0.5 |
| NaCl | 0.33 | 0.33 | 0.33 | 0.33 | 0.33 | 0.33 | 0 | 0 | 0 | 0 | 0 | 0 |
| KCl | 0.06 | 0.06 | 0.06 | 0.06 | 0.06 | 0.06 | 0 | 0 | 0 | 0 | 0 | 0 |

Addition proportion (unit):w/w%

Table 3

| Additive components | Sample No. | | | | | | | | | | | |
|---|---|---|---|---|---|---|---|---|---|---|---|---|
| | 25 | 26 | 27 | 28 | 29 | 30 | 31 | 32 | 33 | 34 | 35 | 36 |
| Px 407 | - | - | - | - | - | 0.5 | - | - | - | - | - | - |
| Tetronic 1107 | - | - | - | - | - | - | 0.5 | - | - | - | - | - |
| PBC-34 | - | - | - | - | - | - | - | 0.5 | - | - | - | - |
| Tween 80 | - | - | - | - | - | - | - | - | 0.5 | - | - | 0.1 |
| NP-18TX | - | - | - | - | - | - | - | - | - | 0.5 | - | - |
| Tyloxapol | - | - | - | - | - | - | - | - | - | - | 0.5 | - |
| ε-aminocaproic acid | 0 | 0.1 | 0.5 | 1 | 3 | 0 | 0 | 0 | 0 | 0 | 0 | 0 |
| HPMC 2910 | 0.15 | 0.15 | 0.15 | 0.15 | 0.15 | 0.15 | 0.15 | 0.15 | 0.15 | 0.15 | 0.15 | 0.15 |
| EDTA·2Na | 0.05 | 0.05 | 0.05 | 0.05 | 0.05 | 0.05 | 0.05 | 0.05 | 0.05 | 0.05 | 0.05 | 0.05 |
| Potassium sorbate | 0.15 | 0.15 | 0.15 | 0.15 | 0.15 | 0.15 | 0.15 | 0.15 | 0.15 | 0.15 | 0.15 | 0.15 |
| Boric acid | 0.5 | 0.5 | 0.5 | 0.5 | 0.5 | 0.5 | 0.5 | 0.5 | 0.5 | 0.5 | 0.5 | 0.5 |
| NaCl | 0.5 | 0.48 | 0.42 | 0.33 | 0 | 0.5 | 0.5 | 0.5 | 0.5 | 0.5 | 0.5 | 0.5 |
| KCl | 0.1 | 0.1 | 0.08 | 0.06 | 0 | 0.1 | 0.1 | 0.1 | 0.1 | 0.1 | 0.1 | 0.1 |

Addition proportion (unit):w/w%

Test on cleaning effect over lipids

The cleaning effect of the ophthalmic composition of the present invention over lipids was examined by means of a lipid-solubilizing rate method. Specifically, a colored lipid was prepared by mixing triglyceride (pharmasol B-112, manufactured by NOF CORPORATION) as a lipid and Sudan I (maximum absorption wavelength: 485.5 nm, manufactured by Nacalai Tesque Inc.) in a weight ratio of 99:1, 0.5 g thereof was weighed by a continuously adjustable pipet (100-1,000 μℓ) and accommodated in a predetermined test bottle, 15 mℓ of the above-obtained sample solution was further added and accommodated in the test bottle, and the opening of the test bottle was covered with a proper lid. This operation was carried out with respect to each of the above sample solutions (Nos. 1-24 and Nos. 30-35).

Then, each test bottle having the colored lipid and the sample solution accommodated therein thus prepared, was shaken at a temperature of 25°C for 24 hours at a constant rate and further left to stand for a predetermined time, and a supernatant fluid in each test bottle was collected, and the absorbance at a wavelength of 485.5 nm was measured with respect to each supernatant fluid by means of a spectrophotometer (recording spectrophotometer UV-2400PC, manufactured by Shimadzu Corporation). The sample solutions were classified into six groups depending upon the type of the surfactant added, and taking the absorbances of samples containing no ε-aminocaproic acid (sample Nos. 30, 31, 32, 33, 34 and 35) in the respective groups as 1, the ratio (absorbance ratio) of the absorbance of each sample solution was calculated, and the results are shown in the following Tables 4 and 5. A value of the absorbance ratio higher than 1, i.e. a relatively high absorbance indicates an excellent cleaning effect over lipids, specifically excellent lipid-solubilizing power.

Table 4

| Surfactant | Sample No. | Concentration of ε-aminocaproic acid (w/w%) | Absorbance ratio |
|---|---|---|---|
| Px407 | 30 | 0 | 1.00 |
| | 1 | 0.1 | 1.03 |
| | 7 | 0.5 | 1.10 |
| | 13 | 1 | 1.15 |
| | 19 | 3 | 1.41 |
| Tetronic 1107 | 31 | 0 | 1.00 |
| | 2 | 0.1 | 1.02 |
| | 8 | 0.5 | 1.07 |
| | 14 | 1 | 1.20 |
| | 20 | 3 | 1.52 |
| PBC-34 | 32 | 0 | 1.00 |
| | 3 | 0.1 | 1.06 |
| | 9 | 0.5 | 1.08 |
| | 15 | 1 | 1.03 |
| | 21 | 3 | 1.04 |

Table 5

| Surfactant | Sample No. | Concentration of ε-aminocaproic acid (w/w%) | Absorbance ratio |
|---|---|---|---|
| Tween 80 | 33 | 0 | 1.00 |
| | 4 | 0.1 | 0.81 |
| | 10 | 0.5 | 0.90 |
| | 16 | 1 | 0.87 |
| | 22 | 3 | 0.82 |

Table 5 (continued)

| Surfactant | Sample No. | Concentration of ε-aminocaproic acid (w/w%) | Absorbance ratio |
|---|---|---|---|
| NP-18TX | 34 | 0 | 1.00 |
| | 5 | 0.1 | 0.99 |
| | 11 | 0.5 | 1.01 |
| | 17 | 1 | 1.09 |
| | 23 | 3 | 1.00 |
| Tyloxapol | 35 | 0 | 1.00 |
| | 6 | 0.1 | 0.96 |
| | 12 | 0.5 | 0.85 |
| | 18 | 1 | 1.07 |
| | 24 | 3 | 1.01 |

As evident from the results shown in Table 4, it was confirmed that each of the sample solutions comprising both polyoxyalkylene block copolymer or its derivative and ε-aminocaproic acid as a caproic acid derivative, showed high lipid-solubilizing power as compared with the sample solution wherein the polyoxyalkylene block copolymer alone was used and no ε-aminocaproic acid was used together. On the other hand, as evident from the results shown in Table 5, it was confirmed that each of the sample solutions comprising a surfactant which did not correspond to a polyoxyalkylene block copolymer or its derivative, showed lipid-solubilizing power at the same or lower level as the case where such a surfactant was used alone, even though ε-aminocaproic acid was used together. From these findings, it is estimated that the lipid-solubilizing power of the polyoxyalkylene block copolymer or its derivative itself can synergistically be increased by incorporating at least one type of caproic acid and its derivatives together with the polyoxyalkylene block copolymer or its derivative in a solvent.

Test on adjustability to lenses

To examine the adjustability of the ophthalmic composition of the present invention to contact lenses, the following test was carried out. Namely, a plurality of commercially available soft contact lenses (Menicon Soft 72, manufactured by Menicon Co., Ltd., lens diameter: 13.5 mm) were prepared, and such lenses were soaked in physiological saline (ISO No. 10344). kept at a temperature of 25°C, and the lens diameters of the contact lenses in a soaked state were measured by means of a projector (universal projector manufactured by Nikon Corporation), and the obtained measured values were recorded as initial values of the lens diameters.

Then, the contact lenses of which the initial values of the lens diameters were thus obtained were soaked in each of the sample solutions (sample Nos. 13-19, 25, 30-35) prepared as mentioned above and physiological saline at a temperature of 25°C for 7 days, and the lens diameters were measured in a soaked state by means of the same projector as mentioned above. This operation was carried out by using three contact lenses with respect to each sample solution.

Then, the difference (d) between the measured value of the lens diameter thus obtained (value after the soaking) and the initial value of the lens diameter was obtained in accordance with the following formula:

$$d=(\text{value after the soaking})-(\text{initial value})$$

with respect to each contact lens, and the average value of the obtained values was obtained with respect to each sample solution. The results are shown in the following Table 6 as the change in lens diameter. Needless to say, the smaller the absolute value of the change in lens diameter, the more excellent the adjustability to contact lenses, and further, the value of the change in lens diameter is particularly preferably within ±0.2 mm.

Table 6

| Sample No. | Change in lens diameter (mm) |
|---|---|
| 13 | +0.063 |

Table 6 (continued)

| Sample No. | Change in lens diameter (mm) |
|---|---|
| 14 | +0.013 |
| 15 | +0.094 |
| 16 | +0.100 |
| 17 | +0.381 |
| 18 | +0.150 |
| 19 | -0.087 |
| 25 | +0.150 |
| 30 | +0.153 |
| 31 | +0.150 |
| 32 | +0.190 |
| 33 | +0.206 |
| 34 | +0.450 |
| 35 | +0.231 |
| ISO* | 0.000 |

*ISO: Physiological saline defined in ISO 10344

As evident from the results shown in Table 6, it was confirmed that each of the sample solutions (sample Nos. 13-15) comprising both polyoxyalkylene block copolymer or its derivative and ε-aminocaproic acid had no influence over the shape (diameter) of contact lenses as compared with the sample solutions (sample Nos. 16-18, 33-35) wherein the surfactant which did not correspond to the polyoxyalkylene block copolymer or its derivative was used alone or in combination with ε-aminocaproic acid. Accordingly, it is understood that the ophthalmic composition of the present invention is advantageous in view of the adjustability to contact lenses, and that it has no harmful influence over contact lenses.

Test on protein deposit inhibitory effect

The following test was carried out to examine the effect of inhibiting protein deposit on contact lenses by the ophthalmic composition of the present invention. Specifically, with respect to the sample solutions 19, 30 and 36, the amount of protein deposit on the contact lens "Pure Vision" manufactured by Bausch&Lomb was measured in accordance with "Protocol for Testing Protein Deposit Inhibition" as disclosed in U.S.P. 6,037,328 and is shown in the following Table 7. Needless to say, the smaller the amount of the protein deposit, the more excellent the effect of inhibiting protein deposit on contact lenses.

Table 7

| Sample No. | Amount of protein deposit (μg/mℓ) |
|---|---|
| 19 | 17.865 |
| 30 | 26.231 |
| 36 | 34.212 |

As evident from Table 7, it was confirmed that the sample solution No. 19 comprising both Poloxamer 407 as a polyoxyalkylene block copolymer and ε-aminocaproic acid as a caproic acid derivative had an excellent effect of inhibiting protein deposit on contact lenses as compared with the sample No. 30 comprising Poloxamer 407 alone or the sample No. 36 comprising Tween 80 as a surfactant which did not correspond to the polyoxyalkylene block copolymer or its derivative alone.

As evident from the above explanation, the ophthalmic composition of the present invention contains as the component A at least one member selected from the group consisting of polyoxyalkylene block copolymers and their derivatives, and further contains as the component B at least one member selected from the group consisting of caproic

acid and its derivatives, whereby the cleaning power of the specific component A is effectively increased, and accordingly excellent cleaning effect over a stain of e.g. lipids on the eye or a contact lens can be realized.

Further, the polyoxyalkylene block copolymer or its derivative as the component A is less likely to irritate the eye relative to other surfactants, and accordingly both the above-described excellent cleaning effect and high safety for the eye can be realized with the ophthalmic composition of the present invention containing the above component A.

## Claims

1. An ophthalmic composition which contains a component A consisting of at least one member selected from the group consisting of polyoxyalkylene block copolymers and their derivatives, and a component B consisting of at least one member selected from the group consisting of caproic acid and its derivatives.

2. The ophthalmic composition according to Claim 1, wherein the component A is a polyoxyethylene-polyoxypropylene block copolymer or its derivative.

3. The ophthalmic composition according to Claim 2, wherein the polyoxyethylene-polyoxypropylene block copolymer or its derivative has a HLB value of from 2 to 40.

4. The ophthalmic composition according to any one of Claims 1 to 3, wherein the component A is contained in a proportion of from 0.01 to 5.0 w/w%.

5. The ophthalmic composition according to any one of Claims 1 to 4, wherein the component B is contained in a proportion of from 0.01 to 5.0 w/w%.

6. The ophthalmic composition according to any one of Claims 1 to 5, wherein at least one member selected from the group consisting of a preservative, a thickener, a buffering agent, a chelating agent, an isotonicity agent and a surfactant is further contained.

7. An ophthalmic solution which comprises the ophthalmic composition as defined in any one of Claims 1 to 6.

8. An ophthalmic solution which comprises the ophthalmic composition as defined in any one of Claims 1 to 6, which is used for cleaning a contact lens put on the eye by administering the solution to the eye.

9. An agent for contact lenses which comprises the ophthalmic composition as defined in any one of Claims 1 to 6.

10. A cleaning solution for contact lenses which comprises the agent for contact lenses as defined in Claim 9, which is used for cleaning a contact lens taken off from the eye by bringing the contact lens into contact with the cleaning solution.

European Patent Office

# EUROPEAN SEARCH REPORT

Application Number
EP 02 02 3548

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 0 938 903 A (ROHTO PHARMA) 1 September 1999 (1999-09-01) * paragraphs [0015]-[0018],[0026]; claims 1,5,6; examples * | 1-4,6-10 | C11D1/722 C11D1/44 C11D3/20 C11D3/33 |
| A | US 4 410 442 A (STONE ROGER L ET AL) 18 October 1983 (1983-10-18) * column 5, line 61 - column 6, line 14; claims 1,10,13 * | 1-10 | |
| A | US 5 814 655 A (BOWMAN LYLE M ET AL) 29 September 1998 (1998-09-29) * claims 1,2; tables 4,5 * | 1-10 | |
| A | DE 28 54 278 A (THILO & CO GMBH DR) 3 July 1980 (1980-07-03) * page 3, paragraphs 1,2; claims 1,2 * | 1-10 | |
| | | | TECHNICAL FIELDS SEARCHED (Int.Cl.7) |
| | | | C11D |

The present search report has been drawn up for all claims

| Place of search | Date of completion of the search | Examiner |
|---|---|---|
| MUNICH | 15 January 2003 | Pentek, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 02 02 3548

This annex lists the patent family members relating to the patent documents cited in the above-mentioned European search report.
The members are as contained in the European Patent Office EDP file on
The European Patent Office is in no way liable for these particulars which are merely given for the purpose of information.

15-01-2003

| Patent document cited in search report | | Publication date | Patent family member(s) | | | Publication date |
|---|---|---|---|---|---|---|
| EP 0938903 | A | 01-09-1999 | AU | 1558597 | A | 28-08-1997 |
| | | | EP | 0938903 | A1 | 01-09-1999 |
| | | | WO | 9728827 | A1 | 14-08-1997 |
| US 4410442 | A | 18-10-1983 | AR | 231802 | A1 | 29-03-1985 |
| | | | AT | 15447 | T | 15-09-1985 |
| | | | AU | 549420 | B2 | 23-01-1986 |
| | | | AU | 1031983 | A | 21-07-1983 |
| | | | BR | 8300127 | A | 04-10-1983 |
| | | | CA | 1186194 | A1 | 30-04-1985 |
| | | | DE | 3266255 | D1 | 17-10-1985 |
| | | | DK | 12183 | A | 14-07-1983 |
| | | | EP | 0083820 | A1 | 20-07-1983 |
| | | | ES | 8406882 | A1 | 16-11-1984 |
| | | | FI | 830104 | A | 14-07-1983 |
| | | | GR | 81931 | A1 | 12-12-1984 |
| | | | IE | 54499 | B1 | 25-10-1989 |
| | | | JP | 58171499 | A | 08-10-1983 |
| | | | NZ | 203015 | A | 13-12-1985 |
| | | | PH | 18951 | A | 14-11-1985 |
| | | | ZA | 8300221 | A | 26-10-1983 |
| US 5814655 | A | 29-09-1998 | NONE | | | |
| DE 2854278 | A | 03-07-1980 | DE | 2854278 | A1 | 03-07-1980 |

EPO FORM P0459

For more details about this annex : see Official Journal of the European Patent Office, No. 12/82